# FASCICULE DE BREVET EUROPEEN

(11) **EP 1 800 683 B1**
(45) Date de publication et mention de la délivrance du brevet: **17.11.2010**
(21) Numéro de dépôt: 06291991.5
(22) Date de dépôt: 20.12.2006
(51) Int. Cl.: A61K 31/55, A61K 31/4422, A61P 9/10

(54) **Nouvelle association d'un inhibiteur du courant if sinusoidal et d'un inhibiteur calcique et les compositions pharmaceutiques qui la contiennent**
Neue Zusammenstellung eines sinusförmigen Stromhemmer und eines Calcium-Kanal-Blocker und deren enhaltende pharmazeutische Zubereitungen
New association of an If sinusoidal current inhibitor and a calcium channel-blocking agent, and pharmaceutical ingredients containing said association

(30) Priorité: 21.12.2005 FR 0513008
(43) Date de publication de la demande: 27.06.2007
(73) Titulaire: Les Laboratoires Servier, 92415 Courbevoie Cedex (FR)
(72) Inventeur: Benatar, Vidal, 92210 Saint-Cloud (FR); Lerebours-Pigeonniere, Guy, 92300 Levallois-Perret (FR)

(56) Documents cités:
- TARDIF J-C: "Ivabradine in clinical practice: Benefits of I(f) inhibition." EUROPEAN HEART JOURNAL, SUPPLEMENT, VOL. 7, NO. H, PP. H29-H32. . REFS: 26 ISSN: 1520-765X CODEN: EHJSFT, 2005, XP002403310
- LOPEZ-BESCOS ET AL.: "Long term safety and antianginal efficacy of the If current inhibitor ivabradine" EUR. HEART J., vol. 25(supl), 2004, page 138, XP009073736
- RUZYLLO ET AL.: "Antianginal and antiischemic effects of the If current inhibitor ivabradine..." EUR. HEART J., vol. 25(supl), 2004, page 138, XP009073742
- GIBBONS ET AL.: "ACC/AHA 2002 guideline update for the management of patients with chronic stable angina-summary article" CIRCULATION, vol. 107, 2003, pages 149-158, XP002403311

## Description

La présente invention concerne une nouvelle composition pharmaceutique comprenant un inhibiteur sélectif et spécifique du courant I_{f} sinusal et d'un inhibiteur calcique.
Plus particulièrement, la présente invention concerne une nouvelle composition comprenant un inhibiteur sélectif et spécifique du courant I_{f} sinusal qui est l'ivabradine ou 3-{3-[{[(7S)-3,4-diméthoxybicyclo[4.2.0]octa-1,3,5-trién-7-yl]méthyl} (méthyl)amino]propyl}-7,8-diméthoxy-1,3,4,5-tétrahydro-2*H*-3-benzazépin-2-one de formule (I) : 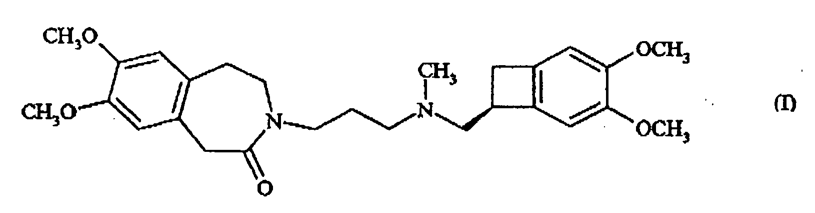 ainsi que ses hydrates, formes cristallines et sels d'addition à un acide pharmaceutiquement acceptable, et d'un inhibiteur calcique et plus particulièrement un inhibiteur calcique de la classe des dihydropyridines.

Les inhibiteurs sélectifs et spécifiques du courant I_{f} sinusal et plus particulièrement l'ivabradine, ainsi que ses hydrates, formes cristallines et ses sels d'addition à un acide pharmaceutiquement acceptable, et plus particulièrement son chlorhydrate, possèdent des propriétés pharmacologiques et thérapeutiques très intéressantes, notamment des propriétés chronotropes négatives (réduction de la fréquence cardiaque), qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0534 859.

La demanderesse a présentement découvert que, de façon surprenante, les inhibiteurs sélectifs et spécifiques du courant I_{f} sinusal et plus particulièrement l'ivabradine ou 3-{3-[{[(7S)-3,4-diméthoxybicyclo[4.2.0]octa-1,3,5-trién-7-yl]méthyl}(méthyl)amino]propyl}-7,8-diméthoxy-1,3,4,5-tétrahydro-2*H*-3-benzazépin-2-one, utilisés en association avec un inhibiteur calcique et plus particulièrement un inhibiteur calcique de la classe des dihydropyridines, possédaient des propriétés tout à fait intéressantes permettant de les utiliser en association dans le traitement de l'angor.

Les inhibiteurs calciques sont des molécules qui ont pour propriété essentielle de bloquer la perméabilité au calcium de certains canaux de la membrane cellulaire. Ils empêchent l'ouverture des pores des canaux voltage-dépendants. Ils s'opposent ainsi à la pénétration du calcium dans les fibres musculaires lisses des vaisseaux. Ils diminuent le taux de calcium libre intracellulaire, ce qui a pour conséquence de réduire le tonus des muscles lisses des vaisseaux périphériques et coronaires. Ainsi, ces composés, et plus particulièrement ceux appartenant à la classe des dihydropyridines sont particulièrement indiqués dans le traitement de l'angor car ils diminuent le retour veineux en soulageant ainsi le travail du ventricule gauche et diminuent la consommation myocardique en oxygène d'une part, et ils améliorent le flux sanguin coronarien grâce à leur action vasodilatatrice au niveau des grosses artères épicardiques d'autre part. Une des conséquences de l'effet vasodilatateur périphérique des dihydropyridines est d'entraîner une tachycardie réflexe qui peut persister chez les patients traités pour angine de poitrine. On connaît la relation forte qui existe entre l'augmentation de la fréquence cardiaque et la mortalité cardiovasculaire chez le coronarien. Ceci est à rapprocher du possible risque d'augmentation de la mortalité cardiovasculaire et des infarctus du myocarde rapportés avec les dihydropyridines.

Les effets indésirables les plus fréquents rencontrés avec les dihydropyridines sont tachycardies, palpitations, céphalées, oedèmes des membres inférieurs dose-dépendants. Il y a donc un besoin réel de nouveaux traitements permettant de bénéficier des effets positifs de ces composés tout en augmentant leur marge de sécurité, particulièrement cardiovasculaire. La publication de Lopez-Bescos et al. (Eur.Heart J.,vol.25,2004) décrit une étude "on top of" consistant à administrer de l' invabradine à des patients déjà traités et stabilisés pour la pathologie angine de poitrine ce qui permet uniquement d'évaluer la sécurité et l' efficacité de l'ivabradine seule.

La demanderesse a présentement découvert que, de façon surprenante, les inhibiteurs sélectifs et spécifiques du courant I_{f} sinusal et plus particulièrement l'ivabradine étaient non seulement capables de potentialiser les effets des inhibiteurs calciques et plus particulièrement ceux appartenant à la classe des dihydropyridines, mais ont montré de plus une excellente capacité à améliorer le profil de sécurité de ces antagonistes calciques, et plus particulièrement les effets indésirables cardiaques, les oedèmes des membres inférieurs et les céphalées. Ce double effet permet d'envisager l'utilisation de la composition selon l'invention dans le traitement de l'angor avec une sécurité d'emploi accrue.

Les inhibiteurs sélectifs et spécifiques du courant I_{f} sinusal dans la composition selon l'invention sont plus particulièrement l'ivabradine, la zatébradine et la cilobradine, ainsi que leurs hydrates, formes cristallines, et sels d'addition à un acide ou à une base pharmaceutiquement acceptable.

Les inhibiteurs caciques dans la composition selon l'invention sont plus particulièrement ceux appartenant à la classe des dihydropyridines. A titre non limitatif, les inhibiteurs calciques dans la composition selon l'invention sont : l'amlodipine, la nifédipine, la félodipine et leurs hydrates, formes cristallines, et sels d'addition à un acide ou à une base pharmaceutiquement acceptable, et plus particulièrement le bésylate ou le maléate.

Plus particulièrement, l'invention concerne une composition comprenant l'ivabradine ou un de ses hydrates, formes cristallines, et sels d'addition à un acide pharmaceutiquement acceptable et plus particulièrement son chlorhydrate, et un inhibiteur calcique ou un de ses hydrates, formes cristallines, et sels d'addition à un acide pharmaceutiquement acceptable.

Encore plus préférentiellement, l'invention concerne une composition comprenant l'ivabradine ou un de ses hydrates, formes cristallines, et sels d'addition à un acide pharmaceutiquement acceptable et plus particulièrement son chlorhydrate, et l'amlodipine ou un de ses hydrates, formes cristallines, et sels d'addition à un acide pharmaceutiquement acceptable et plus particulièrement son bésylate ou maléate.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc... ainsi que les compositions pharmaceutiques avec libération programmée, retardée, prolongée ou différée.

Outre l'inhibiteur sélectif et spécifique du courant I_{f} sinusal et l'inhibiteur calcique, les compositions pharmaceutiques selon l'invention contiennent un ou plusieurs excipients ou véhicules choisis parmi des diluants, des lubrifiants, des liants, des agents de désintégration, des absorbants, des colorants, des édulcorants, etc...

A titre d'exemple et de manière non limitative, on peut citer :
◆ *pour les diluants :* le lactose, le dextrose, le sucrose, le mannitol, le sorbitol, la cellulose, la glycérine,
◆ *pour les lubrifiants :* la silice, le talc, l'acide stéarique et ses sels de magnésium et de calcium, le polyéthylène glycol,
◆ *pour les liants :* le silicate d'aluminium et de magnésium, l'amidon, la gélatine, la tragacanthe, la méthylcellulose, la carboxyméthylcellulose de sodium et la polyvinylpyrrolidone,
◆ *pour les désintégrants :* l'agar, l'acide alginique et son sel de sodium, les mélanges effervescents.

La posologie utile varie selon le sexe, l'âge et le poids du patient, la voie d'administration, la nature de l'affection et des traitements éventuellement associés et s'échelonne de 1 à 500 mg d'ivabradine par 24 heures et plus préférentiellement de 15 à 20 mg par jour et de manière encore préférée de 5 à 15mg par jour. La dose de l'inhibiteur calcique pourra être moindre que celle utilisée lorsqu'il est administré seul.

Les exemples suivants illustrent l'invention et ne la limitent en aucune façon.

### Compositions pharmaceutiques :

### Formule de préparation pour 1000 comprimés dosés à 10 mg d'ivabradine et 5 mg d'amlodipine

:

| | |
|---|---|
| Ivabradine, chlorhydrate | 10 g |
| Amlodipine, bésylate | 5g |
| Lactose monohydrate | 62 g |
| Stéarate de Magnésium | 1,3 g |
| Povidone | 9 g |
| Silice colloïdale anhydre | 0,3 g |
| Cellulose sodium glycolate | 30 g |
| Acide stéarique | 2,6 g |

D'autres exemples de compositions pharmaceutiques selon l'invention sont donnés ci-dessous, à titre non limitatif :

### Exemple 1

| Constituants | Quantité (mg) |
|---|---|
| ivabradine | 10 |
| amlodipine | 5 |

### Exemple 2

| Constituants | Quantité (mg) |
|---|---|
| ivabradine | 15 |
| amlodipine | 5 |

### Exemple 3

| Constituants | Quantité (mg) |
|---|---|
| ivabradine | 10 |
| amlodipine | 10 |

### Exemple 4

| Constituants | Quantité (mg) |
|---|---|
| ivabradine | 15 |
| amlodipine | 10 |

La posologie pour les compositions pharmaceutiques décrites supra consiste en une administration per os d'un comprimé par 24 heures.

Dans les populations à risque correspondant à des patients hypertendus et âgés de plus de 75 ans, la dose critique initiale administrée par voie orale est de 5mg d'ivabradine et 5mg d'amlodipine par 24 heures sous la forme d'un comprimé.

### Etude clinique :

• Deux études cliniques réalisées chez des patients traités par des antagonistes calciques de type dihydropyridine qui se plaignaient toujours de crises douloureuses d'angine de poitrine (malgré l'antagoniste calcique) ont montré qu'un traitement concomitant avec l'ivabradine a permis de diminuer de façon très substantielle celles-ci (environ 60 %).

**Tableau 1 :**

| ***Evolution du nombre de crises angineuses chez des patients recevant des dihydropyridines à l'inclusion et ayant reçu de l'ivabradine pendant 1 an.*** | | | |
|---|---|---|---|
| ***Nombre de crises d'angor de poitrine (par semaine)*** | | | |
| | A l'inclusion | Après traitement | % |
| **Etude 019 :** | | | |
| n = 27 | 1,9 ± 2,8 | 0,7 ± 1,3 | -61,4 |
| **Etude 021 :** | | | |
| n = 114 | 2,2 ± 3,4 | 0,9 ± 2,9 | -58,9 |

| | | | |
|---|---|---|---|
| n = nombre de patients | | | |

• Par ailleurs, de façon surprenante l'association de l'ivabradine à l'amlodipine a entraîné une amélioration du profil de sécurité et d'acceptabilité de l'amlodipine. En effet, dans le cadre du développement clinique de l'ivabradine dans le traitement de l'angine de poitrine; des études sur l'acceptabilité d'ivabradine ont été conduites en comparaison à l'amlodipine en monothérapie ou en association avec l'ivabradine. Les résultats montrent que lorsque l'on associe l'ivabradine à l'amlodipine, la sécurité d'emploi, en particulier cardiaque de celle-ci augmente :

**Tableau 2 :**

| ***Evènements indésirables chez des patients coronariens traités avec amlodipine seule ou avec l'association amlodipine* + *ivabradine par 100 patients-année d'exposition*** | | | |
|---|---|---|---|
| | | Ivabradine + antagonistes calciques n = 686 Patients-année : 262,6 | Amlodipine seule n = 441 Patients-année: 94,8 |
| **Evènements indésirables cardiaques** | | **40,0** | **58,0** |
| | **- Arythmies cardiaques** | 28,6 | 35,9 |
| | **- Angor instable** | 2,3 | 5,3 |
| | **Infarctus du myocarde** | 1,9 | 3,2 |
| | **- Aggravation de la maladie coronaire** | 0,4 | 2,1 |
| | **- Palpitations** | 1,1 | 3,1 |
| **Oedèmes des membres inférieurs** | | 22,9 | 33,5 |
| **Céphalées** | | 3,8 | 9,5 |

| | | | |
|---|---|---|---|
| n = nombre de patients | | | |

Il apparaît clairement un taux plus faible d'événements indésirables lorsque l'ivabradine est ajoutée à l'amlodipine particulièrement pour les événements cardiaques à type d'arythmies cardiaques et d'évènements ischémiques coronariens (angor instable, infarctus du myocarde et aggravation de la maladie coronaire). Il est important de noter que l'incidence des oedèmes des membres inférieurs qui sont pour l'amlodipine l'effet indésirable le plus fréquent et la cause d'arrêt de traitement dans près de 10% des cas diminue de façon très marquée lorsque l'on ajoute ivabradine, ainsi que pour les céphalées.

## Revendications

1. Composition pharmaceutique comprenant un inhibiteur sélectif et spécifique du courant sinusal et un inhibiteur calcique.

2. Composition pharmaceutique selon la revendication 1 dans laquelle l'inhibiteur sélectif et spécifique du courant I_{f} sinusal est l'ivabradine ou 3-{3-[{[(7S-)-3,4-diméthoxybicyclo[4.2.0]octa-1,3,5-trién-7-yl]méthyl}(méthyl)amino]propyl}-7,8-di-méthoxy-1 ,3,4,5-tétrahydro-2*H*-3-benzazepin-2-one, ou un de ses hydrates, formes cristallines ou sels d'addition à un acide pharmaceutiquement acceptable.

3. Composition pharmaceutique selon la revendication 1 dans laquelle l'inhibiteur sélectif et spécifique du courant 1, sinusal est l'ivabradine ou 3-{3-[{[(7S)-3,4-diméthoxybicyclo[4.2.0]octa-1,3,5-trién-7-yl]méthyl}(méthyl)amino]propyl}-7,8-di-méthoxy 1,3,4,5-tétrahydro-2*H*-3-benzazépin-2-one, chlorhydrate ou un de ses hydrates ou formes cristallines.

4. Composition pharmaceutique selon la revendication 1 dans laquelle l'inhibiteur calcique appartient à la classe des dihydropyridines.

5. Composition pharmaceutique selon la revendication 1 dans laquelle l'inhibiteur calcique est l'amlodipine, ou un de ses hydrates, formes cristallines ou sels d'addition à un acide pharmaceutiquement acceptable.

6. Composition pharmaceutique selon la revendication 1 dans laquelle l'inhibiteur calcique est l'amlodipine, bésylate ou un de ses hydrates ou formes cristallines.

7. Composition pharmaceutique selon la revendication 1 qui contient l'ivabradine ou un de ses hydrates, formes cristallines ou sels d'addition à un acide pharmaceutiquement acceptable, et l'amlodipine ou un de ses hydrates, formes cristallines ou sels d'addition à un acide pharmaceutiquement acceptable.

8. Composition pharmaceutique selon la revendication 1 qui contient l'ivabradine, chlorhydrate ou un de ses hydrates ou formes cristallines, et l'amlodipine bésylate ou un de ses hydrates ou formes cristallines.

9. Compositions pharmaceutiques selon l'une des revendications 1 à 8 comprenant un ou plusieurs excipients pharmaceutiquement acceptables.

10. Utillsation d'une composition pharmaceutique selon l'une des revendications 1 à 9 pour l'obtention de compositions pharmaceutiques destinées au traitement de l'angor.

11. Composition pharmaceutique selon l'une des revendications 1 à 9 pour utilisation dans le traitement de l'angor.

## Claims

1. Pharmaceutical composition comprising a selective and specific sinus node I_{f} current inhibitor and a calcium inhibitor.

2. Pharmaceutical composition according to claim 1, wherein the selective and specific sinus node I_{f} current inhibitor is ivabradine, or 3-{3-[{[(7S)-3,4-dimethoxybicyclo[4.2.0]octa-1,3,5-trien-7-yl]methyl}(methyl)amino]propyl}-7,8-dimethoxy-1,3,4,5-tetrahydro-2*H*-3-benzazepin-2-one, or one of its hydrates, crystalline forms or addition salts with a pharmaceutically acceptable acid.

3. Pharmaceutical composition according to claim 1, wherein the selective and specific sinus node I_{f} current inhibitor is ivabradine - or 3-{3-[{[(7S)-3,4-dimethoxybicyclo[4.2.0]octa-1,3,5-trien-7-yl]methyl}(methyl)amino]propyl}-7,8-dimethoxy-1,3,4,5-tetrahydro-2*H*-3-benzazepin-2-one - hydrochloride or one of its hydrates or crystalline forms.

4. Pharmaceutical composition according to claim 1, wherein the calcium inhibitor belongs to the dihydropyridine class.

5. Pharmaceutical composition according to claim 1, wherein the calcium inhibitor is amlodipine, or one of its hydrates, crystalline forms or addition salts with a pharmaceutically acceptable acid.

6. Pharmaceutical composition according to claim 1, wherein the calcium inhibitor is amlodipine besylate or one of its hydrates or crystalline forms.

7. Pharmaceutical composition according to claim 1, which comprises ivabradine, or one of its hydrates, crystalline forms or addition salts with a pharmaceutically acceptable acid, and amlodipine, or one of its hydrates, crystalline forms or addition salts with a pharmaceutically acceptable acid.

8. Pharmaceutical composition according to claim 1, which comprises ivabradine hydrochloride, or one of its hydrates or crystalline forms, and amlodipine besylate, or one of its hydrates or crystalline forms.

9. Pharmaceutical compositions according to one of claims 1 to 9, comprising one or more pharmaceutically acceptable excipients.

10. Use of a pharmaceutical composition according to one of claims 1 to 8 in obtaining pharmaceutical compositions intended for the treatment of angina.

11. Pharmaceutical composition according to one of claims 1 to 9 for use in the treatment of angina.

## Patentansprüche

1. Pharmazeutische Zubereitung umfassend einen selektiven und spezifischen Inhibitor des Sinusstroms I_{f} und einen Calciumkanal-Inhibitor.

2. Pharmazeutische Zubereitung nach Anspruch 1, worin der selektive und spezifische Inhibitor des Sinusstroms I_{f} Ivabradin oder 3-{3-[{[(7S)-3,4-Dimethoxybicyclo[4.2.0]octa-1,3,5-trien-7-yl]-methyl}(methyl)-amino]-propyl}-7,8-di-methoxy-1,3,4,5-tetrahydro-2*H*-3-benzazepin-2-on oder eines seiner Hydrate, Kristallformen oder Additionssalze mit einer pharmazeutisch annehmbaren Säure ist.

3. Pharmazeutische Zubereitung nach Anspruch 1, worin der selektive und spezifische Inhibitor des Sinusstroms I_{f} Ivabradin oder 3-{3-[{[(7S)-3,4-Dimethoxybicyclo[4.2.0]octa-1,3,5-trien-7-yl]-methyl}(methyl)-amino]-propyl}-7,8-di-methoxy-1,3,4,5-tetrahydro-2*H*-3-benzazepin-2-on, das Hydrochlorid oder eines seiner Hydrate oder Kristallformen davon ist.

4. Pharmazeutische Zubereitung nach Anspruch 1, worin der Calciumkanal-Inhibitor der Klasse der Dihydropyridine angehört.

5. Pharmazeutische Zubereitung nach Anspruch 1, worin der Calciumkanal-Inhibitor Amlodipin ist oder eines seiner Hydrate, Kristallformen oder Additionssalze mit einer pharmazeutisch annehmbaren Säure.

6. Pharmazeutische Zubereitung nach Anspruch 1, worin der Calciumkanal-Inhibitor Amlodipin-Besilat oder eines seiner Hydrate oder Kristallformen ist.

7. Pharmazeutische Zubereitung nach Anspruch 1, welche Ivabratin oder eines seiner Hydrate, Kristallformen oder Additionssalze mit einer pharmazeutisch annehmbaren Säure und Amlodipin oder eines seiner Hydrate, Kristallformen oder Additionssalze mit einer pharmazeutisch annehmbaren Säure enthält.

8. Pharmazeutische Zubereitung nach Anspruch 1, welche Ivabradin-Hydrochlorid oder eines seiner Hydrate oder Kristallformen und Amlodipin-Besylat oder eines seiner Hydrate oder Kristallformen enthält.

9. Pharmazeutische Zubereitungen nach einem der Ansprüche 1 bis 8, umfassend einen oder mehrere pharmazeutisch annehmbare Hilfsstoffe.

10. Verwendung einer pharmazeutischen Zubereitung nach einem der Ansprüche 1 bis 9 zur Herstellung von pharmazeutischen Zubereitungen, die zur Behandlung von Angina pectoris bestimmt sind.

11. Pharmazeutische Zubereitung nach einem der Ansprüche 1 bis 9 für die Verwendung bei der Behandlung von Angina pectoris.
